# EUROPEAN PATENT APPLICATION

(11) **EP 4 066 900 A1**
(43) Date of publication of application: **05.10.2022**
(21) Application number: 20893207.9
(22) Date of filing: 26.08.2020
(51) Int. Cl.: A61Q 1/02, A61K 8/97, C09D 11/00, A61K 8/11

(54) **INK FOR TEMPORARY TATTOOS AND PROCESS FOR PRODUCING IT**

(30) Priority: 26.11.2019 MX 2019014139
(71) Applicant: Alatorre Martin, Carlos Enrique, Jalisco, 45129 (MX)
(72) Inventor: VIGUERAS GUZMÁN, Ana Lilia, Guadalajara, Jalisco, 44230 (MX); SANTANA AYALA, Mario Antonio, Tlajomulco de Zúñiga, Jalisco, 45645 (MX); LÓPEZ VILLASEÑOR, Ricardo, Zapopan, Jalisco, 45080 (MX)
(74) Representative: Plasseraud IP
(86) International application number: PCT/MX2020/000029
(87) International publication number: WO 2021/107752

(57) **Abstract**

The present invention relates to an ink composition for temporary tattoos which can be removed when desired and the process for producing it. The ink is characterised in that it is formulated on the basis of biologically tolerable, natural pigments/dyes and is encapsulated in a carrier made of a material which, upon being disturbed by an external energy, can release the dye in the tissue in order to be absorbed by the body, thereby eliminating the tattoo without a trace.

## Description

### Field of the invention.

The present invention relates to the process of combining natural pigments and carriers for making inks for tattoos, specifically for generating an ink for temporary tattoos.

### Background of the invention.

In the field of body tattoos, the human has traditionally used compositions of inks that are permanently applied to the dermis, and whose elimination is difficult or totally imposible. Therefore, rather painful or expensive elimination methods (laser, dermoabrasion, surgery, etc.) are required but they do not guarantee the total elimination.

The ink used in traditional body tattoos is made of too large particles because metallic oxides are employed in their formulation including: ferrocyanide and ferricyanide (yellow, red, green, blue). Also, organic chemicals are used, namely azo chemicals (orange, brown, yellow, green, violet) and chemicals derived from naphthaline-(red). Carbon (soot or ash) is also used for the black.Other elements that are used as pigments include antimony, arsenic, beryllium, calcium, lithium, setenium and sulfur, which cannot be eliminated or removed by the immune system of the human, therefore, they remain in the dermis forming the tattoo visible through the skin.

Another disadvantage of conventional inks is that they are made with artificial dyes (oxides of iron, mercury, carbon, cadmium, titanium, chromium, lead, etc.), which may be harmful to the body in the event that some nanoparticles are released and housed in the lymph nodes as demonstrated in recent studies. Even if a person decides to remove a common tattoo with the laser method, the particles can lodge in the ganglia, which may represent a serious health problem for him.

The present invention has been developed thinking about people that can be allergic to the aforementioned metals and also for people who do not want a tattoo for a lifetime. In these cases, it is better to get a tattoo made of ink lasting the time the person desires and which can be removed easily and without pain.

In this regard, various ink compositions for temporary tattoos have already been developed. Some of them detected in carrying out a background search are described below:
International Publication No. WO2005105020 relating to a method of preparing a compound for drawing a non-permanent tattoo and a method of using said compound.

The above publication discloses a composition for performing temporary tattoos, which is made from *Genipa americana* plant (Genipap) and which also contains with extracts of grapefruit seeds *(Citrous decumana),* rosemary, olive leaves, vitamin E, citric acid and vegetable origin dyes, in which said dyes can be *Lawsonia inermis, Curcuma longa* (turmeric) and/or mixtures thereof. The dyes allow the tattoo lines to be visualized at the time the composition is applied to the user skin.

Although said composition includes a component *(Curcuma Longa)* to be used by the new composition, the process of preparation and the other components of the new composition are not coincident with said former composition.

Canadian patent No. CA 2727849 relating to "HENNA TATTOOS".

This patent discloses a tattoo template made based on henna ink and, rather than being a composition to be used to directly tattoo, consists in a template with a preselected design that is placed on the skin to adhere the henna to the skin of the user and the tattoo printed thereon.

The tattoo template differs entirely from the new proposal since it does not consists in an ink composition formulated to be injected beneath the skin but rather a simple template for previously designed tattoos. In addition, the new proposed composition does not comprise henna material.

As will be evident, the following description of the new ink composition for temporary tattoos is an alternative based on innovative elements and methods.

### OBJECTS OF THE INVENTION

The principal object of the present invention is to develop a novel ink composition for temporary tattoos based on natural pigments obtained from plants, animals, bacteria, fungi and algae. The ink is characterized by an indefinite duration and has the possibility of removing the tattoo when desired by a simple, non invasive treatment that results in complete removal of said tattoo without scarring. The new ink will provide with the following advantages:
- Use of natural components as raw material for obtaining dyes.
- Due to the nature of the employed dyes and their particle size, the practiced tattoo will have a temporary duration.
- Use of biologically tolerable dyes that do not significantly represent a known risk in the organism.
- Neutralizing the presence of toxic compounds that can cause adverse reactions to the user.
- Developing an ink that can be removed by less invasive and more effective methods once applied to the skin.
- Make use of the new encapsulation technologies to provide a carrier for the natural dyes to extend the permanence of the ink and allow for easy removal.
- It Is another object of the present invention to provide a process for removing a tattoo that disappears when an exogenous energy is applied thereon.

### BRIEF DESCRIPTION OF THE DRAWINGS

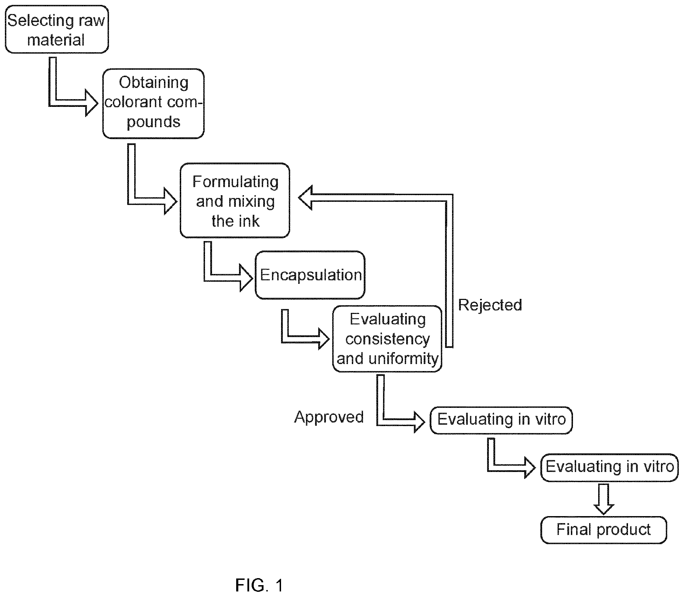

Figure 1 is a schematic view of the process for making the ink for temporary tattoo.

### DETAILED DESCRIPTION OF THE PREFERRED EMBODIMENT OF THE INVENTION

The invention contemplates the creation of a tattoo ink comprising the combination of a pigment or dye with a carrier, in such a way as to control and allow the retention of pigment in the tissue, thereby improving the feeling of temporality.

The new pigments or dyes will have the physical and chemical characteristics necessary to be retained in the tissues and may be associated with carriers to produce a pigment-carrier complex to be retained in the dermis. Said complexes will be fully innocuous and biologically tolerable to the user.

In order to produce temporary tattoos, the pigment should be of natural origin and has a particle size small enough to be absorbed by the body.

The tattoo carriers will comprise a material that when disturbed by an external energy (thermal, sonic, electrical, magnetic, chemical, enzymatic, mechanical, combinations thereof or any other type of energy or combination) may release the pigment or dye into the tissue to be absorbed by the body.

The carriers of the tattoo pigment are biologically tolerated and form pigment-carrier complexes sufficiently large or immunoprotected to be naturally disposed of by the body.

### Pigments.

The invention relates to the use of natural dyes for the formulation of a tattoo ink which, in combination with a carrier, will have the ability to remain indefinitely in the dermis.

The dyes must be obtained from natural sources resulting in natural compounds, preferably but without limitation, as those belonging to the family of carotenoids, quinones, anthraquinones, phenolic compounds, phycobiliproteins, as well as the chemical derivatives thereof by synthesis, substitution, ionic, double substitution, oxidation, reduction, neutralization, decomposition, etc. as well as combinations of said dyes.

Natural coloring agents used for foods, drugs and cosmetics are also contemplated in the preparation of inks.

These pigments may be retained in the dermis by trapping, embedding, complexing or encapsulating in the tattoo pigment carrier. The pigment-carrier complex has a visible color, as well as the physical and chemical characteristics to be retained indefinitely in the dermis in a manner similar to conventional tattoo pigments.

Thus, any naturally colored pigment or substance can be combined with a carrier to form a complex that can be used to produce a tattoo.

### Carriers

According to the present invention the pigments or dyes are trapped, embedded, embedded, complexed or encapsulated in microstructures that act as matrices (tattoo pigment carriers) to form pigment-carrier complexes.

The complexes form an ink that can be used in any conventional tattoo process. They can be formulated so that they remain indefinitely in the dermis and then disappear, or which can be eliminated by the imposition of an exogenous force, which will provide the effect of temporality of the ink once applied to the skin.

Techniques for carrying out microencapsulation include spray drying, extrusion, fluidized bed, simple or complex coacervation, liposomes, inclusion in complexes, spray coating, interfacial polymerization, electrostatic deposition and ionic gelation.

The carriers may comprise any substance that is biologically tolerated and have the size sufficient to contain the pigment/dye particles and may be, but are not limited to, microcapsules, microspheres, polyamide microcapsules, liposomes, cyclodextrins, alginate/alginic acid, polyacrylamides, waxes, cellulose derivatives, chitin, chitosan, etc. as well as combinations thereof.

The carrier carries a colored pigment or colorant to be applied to the dermis, and is sufficiently transparent and translucent that allows the color of the pigment or dye to be displayed across the skin and visible.

The carrier materials are selected so that the pigment-carrier complexes spontaneously disappear in a passive manner (dissolution in the interstitial fluid) or in an active manner (immune system processes), after a certain period of time or being susceptible to an externally applied power source.

The particle size supported by the carrier will be between 5-800 microns, sufficient to contain the colorant within the matrix.

### Additives

An additive will be all substance forming part of the formula and which is intended to help stabilize, mix, emulsify and preserve the main ingredients of the tattoo ink. The compounds will have no negative effect on the health of the user because of their innocuous chemical nature.

### Preservatives

The formulation will contain substances which, introduced into a complex system, will allow the physicochemical properties of each component to be maintained for the longer time to ensure the microbiological quality. In addition to preserving the mixture and preventing degradation of the main components of the ink, any pathological reaction in the user will be avoided.

These compounds include broad spectrum biocides namely, without limitation, phenoxyethanol, ethylhexyglycerine, sodium benzoate, benzyl alcohol, isopropyl alcohol, sorbitan caprylate, caprylyl glycol, 5-Bromo-5-nitro-1,3-dioxan, Quaternium-15, Diazolidinyl urea+methyl, Methylchloroisozolinone, phenoxyethanol+methyl, ethyl propyl paraben; and antioxidants, as well as combinations thereof.

### Emulsifiers

Chemical compounds that act as surfactants, emulsifiers, stabilizers, lubricants, antiaesthetics or solubilizers to facilitate mixing of the ingredients of the ink.

These compounds include, sodium dodecyl sulfate, polyoxyethylene (20) sorbitan monolaurate, phenyl polyethylene glycol, 3-(dodecyldimethylammonium)-propanesulfonate, (C8-C20) unbranched alkyl groups, (C8-C20) branched alkyl groups, and longer length alkyl groups, resin derivatives, high molecular weight propylene oxide polymers, perfluoroalkyl groups, polysiloxane groups, lignin derivatives and without limitation, all of those resulting from the reaction of castor oil with ethene oxide.

### Method of elimination

In another embodiment of the invention, the tattoos made of the proposed ink can be removed on request, i.e., they can be eliminated when the user so desired.

These semi-permanent tattoo inks are produced by complexing, encapsulating or associating the pigments/dyes with tattoo pigment carriers.

The exposure to the exogenous specific energy modifies the tattoo pigment carrier, thereby releasing the trapped pigment into the matrix to allow spontaneous removal of the pigment from the dermis.

The exogenous force may be thermal, sonic (sonic and ultrasonic) light (laser, infrared, ultraviolet), electrical, magnetic, chemical, enzymatic, mechanical, or a combination of any other type of energies that release the pigment or dye from the carrier.

### Process

The above objects are achieved by a novel method for preparing the ink composition for temporary tattoo, which is characterized generally by the following steps:
1. Selecting the raw material from which the natural colorant will be obtained.
2. Obtaining the natural dye by selecting preferably from the family of the carotenoids, quinones, anthraquinones, phenolic compounds, phycobiliproteins, and their derivatives as well as combinations thereof.
3. Formulating a tattoo ink by taking into account the natural colouring agent, the additives and the carrier.
4. Evaluating the consistency and uniformity of the composite ink.
5. Evaluating in vitro the ink.
6. Evaluating in vivo the formulated ink.
7. Obtaining the final ink for temporary tattoos.

With the above described process, a temporary ink produced with natural elements is obtained, which comprises smaller particles that can be absorbed by the body and which can be agglutinated into a matrix so that they can remain in the skin as if they were a conventional tattoo. When the person decides to eliminate the tattoo made of the new ink, said tattoo can be removed by simply applying an exogenous energy that ruptures the matrix containing the ink particles and they will be easily absorbed by the body.

Within the step of formulating the ink, the natural colorant is mixed with other components such as preservative, lubricant, emulsifier, carrier and water to thereafter proceed to the step of evaluating the mixture.

Afterwards, the evaluation of consistency and uniformity of the mixture is carried out, in which the properties of the mixture resulting from the formulation of the ink are evaluated. Once said mixture has been evaluated, there are two alternatives according to the results obtained by the evaluation of the ink: if said ink does not meet the parameters of consistency and uniformity, the ink is reformulated by applying again and adjusting the natural dye to the components used in the formulation step to obtain the desired characteristics.

Once obtained and approved the required consistency and uniformity characteristics, said mixture passes to the in vitro evaluation step, which consists in experimentation outside of live organisms to ensure safety.

Once approved the in vitro evaluation step, then the in vivo evaluation step is carried out, which consists in the experimentation of the ink within live organisms to ensure safety and discard adverse reactions.

Finally, after performing and approved the in vivo evaluation step, the process of formulating the ink for temporary tattoo culminates with obtaining said ink as the final product.

In a preferred embodiment of the invention, the tattoo ink composition is characterized by comprising:
- 5 to 70% of a natural dye, preferably selected from the family of carotenoids, quinones, anthraquinones, phenolic compounds, phycobiliproteins and their chemical derivatives, as well as combinations thereof; and
- 0.01 to 80% of other additional components (preservative, water, emulsifier, carrier).

Although the invention has been described in the context of the preferred embodiments, it will be evident to one skilled in the art that the scope of the inventive concept described by way of example extends beyond the specifically described process to other possible alternate embodiments that may be deducted or derivable from the principles set forth above. Accordingly, although the invention has been described in detail in its preferred embodiment, it will be inferred that the composition or some steps or conditions of the process, or the aforementioned components may be substituted by other analogous or other modifications which are incorporated in light of the foregoing description without departing from the spirit or nature of the claimed invention.

Accordingly, it is intended that the scope of the protection of the present invention be not interpreted based solely on the above described embodiment but is determined by a reasonable interpretation of the content of the following claims.

The best method for carrying out the invention is that which has been described hereinafter.

## Claims

1. A process for preparing an ink for temporary tattoos, which comprises the following steps:
- selecting the raw material to make the ink based on natural origin pigments;
- obtaining the dye extracted from the selected raw material;
- formulating the ink by mixing the colorant extract with other components to provide it the appropriate characteristics;
- evaluating the consistency and uniformity of the formulated ink to determine if they are suitable and optimum;
- reformulating the ink if the firstly formulated one does not meet the required characteristics;
- evaluating the formulated ink *in vitro* to determine the safety thereof in live organisms; and
- finally, evaluating *in vivo* the formulated ink to determine the safety thereof in humans.

2. The process of claim 1 wherein the coloring material is selected to preferably obtain colorants such as those belonging to the family of carotenoids, quinones, anthraquinones, phenolic compounds, phycobiliproteins; and their derivatives as well as combinations thereof.

3. The process of claim 1 wherein the obtained ink is encapsulated in a carrier suitable for its application.

4. An ink for temporary tattoos, which is made based on natural pigments; said ink being **characterized by** comprising:
- 5 to 70% of a naturally occurring dye, preferably as those belonging to the family of carotenoids, quinones, anthraquinones, phenolic compounds, phycotoxins and their chemical derivatives, as well as combinations thereof.
- 0.01 to 80% of other additional components (preservative, water, emulsifier, carrier, additive).

5. The ink for temporary tattoos of the preceding claim, **characterized in that** said ink can be removed when desired without pain and without leaving scarring, by applying an external energy that releases the pigment to be absorbed by the body.
